# EUROPEAN PATENT APPLICATION

(11) **EP 3 578 267 A1**
(43) Date of publication of application: **11.12.2019**
(21) Application number: 17895415.2
(22) Date of filing: 12.12.2017
(51) Int. Cl.: B03C 1/02

(54) **APPARATUS AND METHOD FOR ISOLATING OR PURIFYING TARGET SUBSTANCE FROM BIOLOGICAL SAMPLE**

(30) Priority: 03.02.2017 KR 20170015513
(71) Applicant: Nanobiosys Inc., Seoul 08511 (KR)
(72) Inventor: KIM, Sung Woo, Seoul 07445 (KR); BYUN, Jae Young, Bucheon-si Gyeonggi-do 14529 (KR)
(74) Representative: Isarpatent
(86) International application number: PCT/KR2017/014546
(87) International publication number: WO 2018/143557

(57) **Abstract**

The present invention relates to an apparatus and method for efficiently isolating or purifying a target substance from a biological sample by using a sample comprising magnetic particles and an apparatus comprising a magnet. In the apparatus according to the present invention, a transfer unit, together with a rotation motion, moves up and down in a reagent inside a chamber, and thus the magnetic particles and a buffer are sufficiently mixed, so that even when a small amount of elution buffer is used, the mixing of the magnetic particles and the buffer can be attained. In addition, since the reagent inside the chamber is mixed by fast and strong rotation, the process does not proceed while the magnetic particles remain at the corner of the chamber or adhere to a wall portion thereof, with the results that the extraction efficiency of the target substance can be maximized and the efficiency of subsequence PCR analysis can also be improved.

## Description

### [Technical Field]

The present invention relates to an apparatus and method for isolating or purifying a target substance from a biological sample, and more particularly, to an apparatus and method which can efficiently isolate or purify a target substance from a biological sample using a unit providing sufficient mixing of a buffer and magnetic particles in a reaction chamber.

### [Background Art]

Apparatuses for isolating or purifying nucleic acids or biological substances from various biological samples such as blood and cells are widely used in various fields, such as biology, biochemistry, molecular medicine, forensic medicine, and diagnostic medicine.

Recently, polymerase chain reaction (PCR) for DNA amplification, which is the foundation of a rapid growth of biotechnology, is frequently used as an essential step in the field of biological research and diagnosis.

Although typical methods for isolation of nucleic acids used in PCR generally use organic solvents such as phenol and chloroform, a material having affinity to nucleic acids is used in several recent methods. Examples of such a material comprise silica particles, glass fibers, anion-exchange resins, and magnetic beads.

US Patent Publication No. 5,234,809A discloses a method for isolating nucleic acid from a biological sample using silica particles, which comprises mixing a nucleic acid-containing starting material with a chaotropic material and a silica-containing solid having affinity to the nucleic acid, separating the solid having the nucleic acid from the mixture, washing the obtained solid-nucleic acid complex, and isolating the nucleic acid from the complex using an elution buffer.

In addition, WO1991/12079 discloses a method for extracting nucleic acid using magnetic beads, which comprises precipitating the nucleic acid through the addition of a salt and an alcohol, aggregating the magnetic beads with the nucleic acid, washing suspended solids out with a nucleic acid-bead aggregate fixed using an external magnetic field, and isolating the nucleic acid through elution.

Such methods using solid materials having affinity to nucleic acids do not require harmful organic solvents, minimize physical and biochemical degradation of nucleic acids during isolation, and reduce the effects of nuclease on fixed nucleic acids, and thus are being still used while been repeatedly improved in various ways.

In particular, automated nucleic acid-extraction apparatuses, capable of eliminating the need to manually move samples using a pipette, have been developed in recent years. Most of these automated devices use magnetic particles to remove a step of using a chemical solvent and a centrifugation step from a process of collecting nucleic acids or biological materials from various biological samples.

However, such a method of isolating or purifying nucleic acids or various biological materials using magnetic particles has a problem in that the magnetic particles are not likely to be sufficiently mixed with a buffer or to be sufficiently washed, causing deterioration in efficiency of extracting target biological materials. In addition, since isolation and extraction of nucleic acids is likely to be carried out with magnetic particles adhering to a side wall of a container, the magnetic particles can flow into a PCR device together with the extracted nucleic acids, causing deterioration in the reliability of PCR analysis results.

### [Detailed Description of the Invention]

### [Technical Problem]

Embodiments of the present invention have been conceived to solve such a problem in the art and it is an aspect of the present invention to provide an apparatus and method for isolating or purifying a target substance from a biological sample, which can sufficiently stir the biological sample in a reaction chamber, thereby improving efficiency in isolating the target substance such as nucleic acid, and can keep magnetic beads clean during the transfer and treatment of the magnetic beads.

### [Technical Solution]

In accordance with one aspect of the present invention, an apparatus for isolating or purifying a target substance from a biological sample using magnetic particles comprises:
a container comprising a plurality of chambers each having an opening at an upper side thereof; and
a transfer unit movable up and down to be introduced into the chambers, partially or entirely formed of a magnet to hold the magnetic particles by magnetic force, and movable between the chambers to transfer the magnetic particles between the chambers.

In one embodiment, the transfer unit may comprise: a magnet portion movable up and down in an axial direction thereof and partially or entirely formed of the magnet; and an outer ring portion movable up and down and between the chambers and allowing the magnet portion to move along a center thereof.

In one embodiment, the transfer unit may further comprise a magnet cover preventing contamination of the magnet.

In one embodiment, the magnet cover may be detachably coupled to the outer ring portion. Here, the magnet cover may be coupled to the outer ring portion through press fitting.

In one embodiment, the outer ring portion may be rotatably connected to a rotary motor and the magnet cover may be rotatable by rotation of the outer ring portion. Here, the outer ring portion and the magnet cover may be rotated at 100 rpm to 4,000 rpm.

In one embodiment, the magnet cover may be formed of a material selected from the group consisting of polycarbonate (PC), polystyrene (PS), polymethylmethacrylate (PMMA), cycloolefin copolymer (COC), polyethylene (PE), polyamide (PA), polypropylene (PP), polyphenylene ether (PPE), polyoxymethylene (POM), polyetheretherketone (PEEK), polytetrafluoroethylene (PTFE), polyvinylchloride (PVC), polyvinylidene fluoride (PVDF), polybutylene terephthalate (PBT), fluorinated ethylene propylene (FEP), perfluoralkoxyalkane (PFA), acrylonitrile-butadiene-styrene (ABS), and combinations thereof.

In one embodiment, the magnet cover may comprise a protrusion portion comprising at least one protrusion.

In one embodiment, the magnet portion may comprise the magnet and a support supporting the magnet and may further comprise an elastic member between the magnet and the support.

In one embodiment, the magnet may be a permanent magnet or an electromagnet.

In one embodiment, the plurality of chambers of the container may be connected to one another in a row, and an angle formed between a wall of each chamber and a plane perpendicular to a bottom of the chamber may range from 3 to 10 degrees.

In one embodiment, the biological sample may be at least one selected from the group consisting of blood, sputum, bacteria, semen, cell tissue, saliva, hair, urine, an aspirate sample, a swab sample, and a cell culture fluid, and the target substance may be nucleic acid.

In one embodiment, the magnetic particles may be formed of a paramagnetic or superparamagnetic material and may comprise an outer silica coating.

In accordance with another aspect of the present invention, a transfer device for transferring magnetic particles from a first chamber to a second chamber comprises: a magnet portion movable up and down in an axial direction thereof and partially or entirely formed of a magnet; and an outer ring portion movable up and down and between the chambers and allowing the magnet portion to move along a center thereof.

In one embodiment, the transfer device may further comprise a magnet cover preventing contamination of the magnet.

In one embodiment, the magnet cover may be detachably coupled to the outer ring portion. Here, the magnet cover may be coupled to the outer ring portion through press fitting.

In one embodiment, the outer ring portion may be rotatable and the magnet cover may be rotatable by rotation of the outer ring portion.

In one embodiment, the magnet cover may comprise a protrusion portion comprising at least one protrusion.

In accordance with a further aspect of the present invention, a method for transferring magnetic particles from a first chamber to a second chamber comprises:
providing a reagent comprising the magnetic particles to the first chamber;
positioning a transfer unit on the first chamber, the transfer unit comprising a magnet portion movable up and down in an axial direction thereof and partially or entirely formed of a magnet, an outer ring portion movable up and down and between the chambers and allowing the magnet portion to move along a center thereof, and a magnet cover detachably coupled to the outer ring portion and preventing contamination of the magnet;
immersing a portion of the magnet cover in the reagent by moving the transfer unit downward with the magnet portion moved upward;
stirring the reagent in the first chamber through rotation, vertical movement, or rotation and vertical movement of the magnet cover;
securing the magnetic particles to a surface of the magnet cover by moving the magnet portion downward; and
moving the transfer unit having the magnetic particles secured thereto to the second chamber.

In one embodiment, the method may further comprise, after moving the transfer unit to the second chamber, releasing the secured magnetic particles by moving the magnet portion upward.

In one embodiment, the reagent may comprise a biological sample retaining a target substance and a lysis buffer, wherein the biological sample may be dissolved in the lysis buffer to discharge the target substance therefrom such that the discharged target substance is coupled to the magnetic particles.

In one embodiment, the magnet cover may comprise a protrusion portion comprising at least one protrusion.

In one embodiment, the biological sample may be at least one selected from the group consisting of blood, sputum, bacteria, semen, cell tissue, saliva, hair, urine, an aspirate sample, a swab sample, and a cell culture fluid, and the target substance may be nucleic acid.

In one embodiment, the magnetic particles may be formed of a paramagnetic or superparamagnetic material and may comprise an outer silica coating.

In accordance with yet another aspect of the present invention, a method for isolating or purifying a target substance from a biological sample using magnetic particles comprises:
dissolving the biological sample and coupling the target substance to the magnetic particles by providing a reagent comprising the biological sample, the magnetic particles, and a lysis buffer to a first chamber;
positioning a transfer unit on the first chamber, the transfer unit comprising a magnet portion movable up and down in an axial direction thereof and partially or entirely formed of a magnet, an outer ring portion movable up and down and between the chambers and allowing the magnet portion to move along a center thereof, and a magnet cover detachably coupled to the outer ring portion and preventing contamination of the magnet;
immersing a portion of the magnet cover in the reagent by moving the transfer unit downward with the magnet portion moved upward;
stirring the reagent in the first chamber through rotation, vertical movement, or rotation and vertical movement of the magnet cover;
securing the magnetic particles having the target substance coupled thereto to a surface of the magnet cover by moving the magnet portion downward;
washing the magnetic particles by moving the transfer unit, to which the magnetic particles having the target substance coupled thereto have been secured, to the second chamber containing a washing buffer; and
releasing the target substance from the magnetic particles by moving the transfer unit having the washed magnetic particles secured thereto to a third chamber containing an elution buffer.

In one embodiment, the method may further comprise, after washing the magnetic particles, further washing the magnetic particles by moving the transfer unit having the washed magnetic particles secured thereto to a fourth chamber containing a second washing buffer.

In one embodiment, the magnet cover may comprise a protrusion portion comprising at least one protrusion.

In one embodiment, the biological sample may be at least one selected from the group consisting of blood, sputum, bacteria, semen, cell tissue, saliva, hair, urine, an aspirate sample, a swab sample, and a cell culture fluid, and the target substance may be nucleic acid.

In one embodiment, the magnetic particles may be formed of a paramagnetic or superparamagnetic material and may comprise an outer silica coating.

### [Advantageous Effects]

In the apparatus according to the present invention, magnetic particles can be thoroughly mixed with a buffer through the rotation and vertical movement of the transfer unit immersed in a reagent in a chamber, whereby mixing of the magnetic particles with the buffer can be achieved even using a small amount of an elution buffer.

In addition, since the reagent in the chamber is stirred through fast and strong rotation of the transfer unit, it is possible to prevent process failure due to magnetic particles staying at the corners of the chamber or adhering to a wall of the chamber, thereby maximizing efficiency in extracting a target substance while allowing a subsequent PCR analysis process to be conducted more efficiently.

### [Brief Description of the Drawings]

FIG. 1 shows a perspective view (a) and a sectional view (b) of a container 100 according to one embodiment of the present invention.
FIG. 2 is a sectional view of a transfer unit 200 according to one embodiment of the invention.
FIG. 3 shows a perspective view of a drive unit 300 according to one embodiment of the present invention.
FIG. 4 shows a perspective view (a), sectional view (b), and partially enlarged view (c) of a magnet cover 230 according to one embodiment of the present invention.
FIG. 5 is a view illustrating a process of transferring magnetic particles between chambers using the transfer unit 200 according to one embodiment of the present invention.

### [Best Mode for Carrying Out the Invention]

Hereinafter, embodiments of the present invention will be described with reference to the accompanying drawings. It should be understood that the present invention is not limited to the following embodiments and may be embodied in different ways, and that the embodiments are provided for complete disclosure and thorough understanding of the present invention by those skilled in the art.

An apparatus according to the present invention is used to isolate or purify a target substance from a biological sample with high efficiency using magnetic particles.

As used herein, the term "biological sample" refers to a sample comprising living cells containing a target substance to be analyzed or detected, and may comprise, for example, blood, sputum, bacteria, semen, cell tissue, saliva, hair, urine, an aspirate sample, a swab sample, and a cell culture fluid.

As used herein, the term "target substance" refers to a substance to be finally analyzed or detected, and may comprise any substance that can be immobilized by solid particles. Accordingly, the target substance may comprise, for example, nucleic acids such as DNA and RNA, protein, polypeptide, bioaffinity components used in mechanical measurement (antibodies, antigens, or complexes thereof), bioaffinity components used as protein tablets (for example, biotinylated protein), streptavidin, and complexes thereof.

As used herein, the term "magnetic particles" refers to fine particles formed of a magnetic or magnetizing material, wherein the magnetic or magnetizing material refers to a paramagnetic material, a superparamagnetic material, or a ferromagnetic material. In one embodiment of the invention, the magnetic particles may be paramagnetic or superparamagnetic particles.

According to the present invention, the magnetic particles may be coated with a hydrophilic material to be coupled to biomaterials such as nucleic acids, and is preferably coated with silica, or a carboxyl group. In the present invention, the magnetic particles may be silica-coated magnetic particles.

Magnetic particles applicable to the present invention may have a particle diameter of 100 nm to 1,000 nm, preferably 200 nm to 800 nm, more preferably 300 nm to 700 nm.

One aspect of the present invention relates to an apparatus for isolating or purifying a target substance from a biological sample using magnetic particles. The apparatus comprises: a container 100 comprising a plurality of chambers 110 each having an opening at an upper side thereof; and a transfer unit 200 movable up and down to be introduced into the chambers, partially or entirely formed of a magnet to hold the magnetic particles (hereinafter, also referred to as "magnetic beads") by magnetic force, and movable between the chambers to transfer the magnetic particles between the chambers.

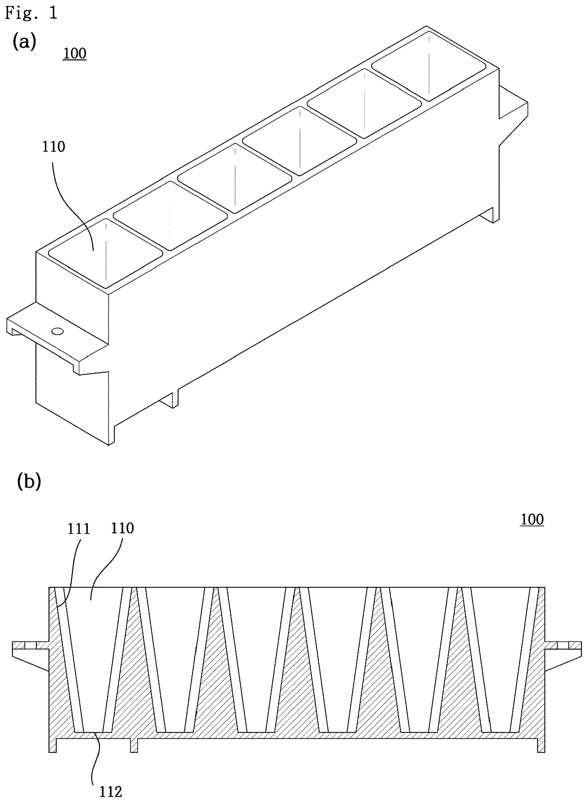

Referring to FIG. 1(a) and FIG. 1(b), the container 100 comprises the plurality of chambers 110, which are preferably arranged in a row. Each of the chambers 110 is provided with a biological sample, magnetic beads, a buffer, or the like suitable for a desired processing procedure. Specifically, after a buffer suitable for each process is provided with a corresponding chamber 110, the magnetic beads are moved between the chambers using the transfer unit 200 according to the present invention, thereby finally isolating the target substance.

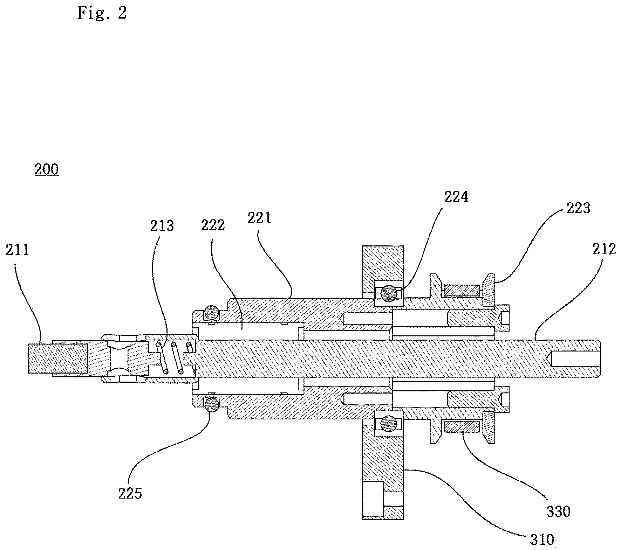

In the present invention, the transfer unit 200 serves to move the magnetic particles, preferably the magnetic particles which form a complex in combination with the target substance, from one chamber to another chamber. FIG. 2 is a sectional view of a transfer unit 200 according to one embodiment of the invention. A transfer unit 200 according to this embodiment is mainly composed of a magnet portion 210 and an outer ring portion 220, and may further comprise a magnet cover 230.

Referring to FIG. 2, the magnet portion 210 is disposed to pass through a central axis of the outer ring portion 220 and is movable up and down in an axial direction thereof. Here, the magnet portion 210 may be moved down to a location at which a lower end of the magnet portion 210 is sufficiently close to a bottom 112 of the chamber 110 to apply magnetic force to the magnetic beads on the bottom 112 of the chamber 110 from a magnet 211 of the magnet portion 210. In addition, the magnet portion 210 may be moved up to a location at which the magnet 211 of the magnet portion 210 applies little or no magnetic force to the magnetic beads in the chamber 110. Herein, the expression "the magnet 211 applies little or no magnetic force to the magnetic beads" means that mixing of the magnetic beads with a buffer is not affected by magnetic force of the magnet.

As described above, the magnet portion 210 comprises the magnet 211 to hold the magnetic particles by magnetic force. Preferably, the magnet 211 is located at the lower end of the magnet portion 210 to apply magnetic force to the magnetic particles on the bottom 112 of the chamber 110. The magnet 211 may be a permanent magnet or an electromagnet, preferably a permanent magnet.

In addition, the magnet portion 210 may comprise a support 212 supporting the magnet 211. The support 212 is disposed inside the outer ring portion 220 to be movable up and down, and transmits vertical movement thereof to the magnet 211. In addition, the support 212 may be connected to a means that is present outside the transfer unit 200 to control the vertical movement of the support. The magnet portion 210 may comprise an elastic member 213 disposed between the magnet 211 and the support 212 to provide impact absorption. Preferably, the elastic member 213 is a spring. Specifically, the elastic member 213 serves to prevent damage to or detachment of the magnet cover due to impact upon coupling of the magnet cover to the magnet.

As shown in FIG. 2, the outer ring portion 220 is configured to allow the magnet portion 210 to pass through the center thereof and is movable onto each chamber such that the magnet portion 210 is located inside the chamber. In addition, the outer ring portion 220 may be movable up and down independently of axial vertical movement of the magnet portion 210. In this way, the transfer unit 200 itself can be introduced into the chamber 110 and thus the magnet cover described below can be moved close to the bottom 112 of the chamber 110.

The outer ring portion 220 may comprise a body 221, a linear bushing 222, and a pulley 223. The pulley 223 transmits torque from the outside to the outer ring portion 220 such that the outer ring portion 220 can be rotated. The linear bushing 222 is disposed between the body 221 and the magnet portion 210 to prevent rotation of the outer ring portion 220 from being transmitted to the magnet portion 210 while allowing vertical movement of the magnet portion 210.

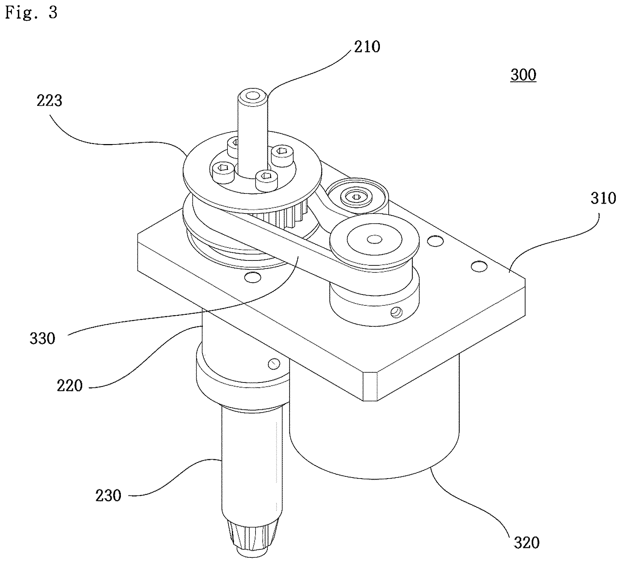

FIG. 3 is a perspective view of a drive unit 300 comprising the outer ring portion 220 and a rotary motor. The outer ring portion 220 is supported on a support plate 310 and receives torque of the rotary motor 320 via a timing belt 330 and the pulley 223. As shown in FIG. 2, a bearing 224 is disposed between the outer ring portion 220 and the support plate 310 such that the outer ring portion 220 supported on the support plate 310 can be smoothly rotated. Preferably, the rotary motor 320 is a 2,000 to 5,000 rpm DC motor.

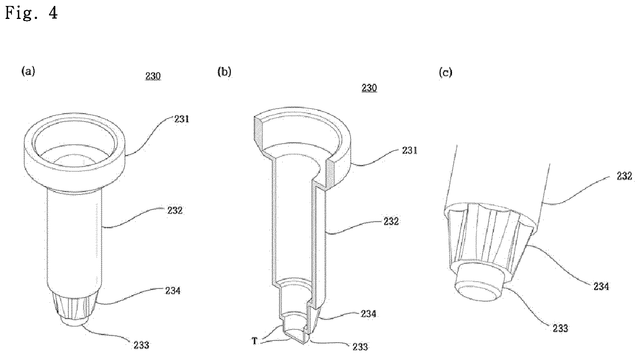

The transfer unit 200 according the present invention may further comprise the magnet cover 230 as shown in FIG. 4. The magnet cover 230 is configured to cover the magnet 211 at the lower end of the magnet portion 210 to prevent the magnet 211 from being contaminated by a reagent. Since the magnet cover 230 directly contacts the reagent, the magnet cover 230 is preferably disposable.

The magnet cover 230 may be detachably coupled to the body 221 of the outer ring portion 220. For example, the magnet cover 230 may be coupled to the body 221 of the outer ring portion 220 through press fitting. In this case, the body 221 may comprise a sealing member, such as an O-ring, which is disposed at a junction between the body 221 and the magnet cover 230 to prevent the reagent from flowing onto the magnet through the junction while allowing firm coupling between the magnet cover 230 and the body 221 and easy detachment of the magnet cover 230 from the body upon removing the magnet cover after use.

In the present invention, since the magnet cover 230 is detachably coupled to the outer ring portion 220, torque of the outer ring portion 220 can be directly transmitted to the magnet cover 230 to rotate the magnet cover. Upon rotating the outer ring portion 220 by operating the rotary motor after moving the outer ring portion 220 downward to allow the magnet cover 230 to be placed in the reagent in the chamber, the magnet cover 230 is capable of being rotated within the reagent in the chamber to stir the reagent. Further, the magnet cover 230 undergoes both vertical movement and rotational movement within the reagent upon operating the outer ring portion 220 to rotate simultaneously with moving up and down, whereby the reagent can be more efficiently stirred. In this way, with the magnet cover 230 providing efficient stirring of the reagent, reactions in the reagent can proceed more actively and the magnetic beads on the bottom can be suspended to be easily secured by a magnetic force in a subsequent process. In addition, particles adhered to the bottom or wall of the chamber can be released I nto the reagent, thereby improving reaction reliability.

The magnet cover 230 may comprise a coupling portion 231, a body portion 232, a particle securing portion 233, and a protrusion portion 234. The coupling portion 231 is adapted to be coupled to the body 221 of the outer ring portion 220 and preferably has a suitable shape for coupling to the body 221 of the outer ring portion 220. For example, if the coupling portion 231 is coupled to the body 221 through press fitting, it is desirable that the coupling portion 231 has an inner diameter suitable for press fitting into the body 221.

The body portion 232 secures a space for vertical movement of the magnet portion 210 and extends a length that allows the magnet 211 at the lower end of the magnet portion 210 to contact an inner lower end of the magnet cover 230 upon moving the magnet portion 210 to a lowermost position thereof.

When the magnet portion 210 is moved to the lowermost position thereof, the magnet 211 is located inside the particle securing portion 233. Accordingly, the magnetic particles can be secured to the particle securing portion 233 by the magnetic force of the magnet 211. Preferably, the particle securing portion 233 has a shape suitable for close contact with the magnet 211 without unnecessary consumption of magnetic force. In addition, the particle securing portion 233 preferably has a thickness T of 2 mm or less, preferably 1 mm or less, more preferably 0.5 mm or less, so as to easily transmit magnetic force therethrough.

According to the present invention, the magnet cover 230 preferably comprises the protrusion portion 234 at a lower end of the body portion 232. Referring to FIG. 4(c), the protrusion portion 234 may comprise at least one protrusion to allow the reagent to be more efficiently stirred upon rotation of the magnet cover 230 in the reagent. Although the shape of the protrusion may be simple as shown in FIG. 4(c), it should be understood that the present invention is not limited thereto, and the protrusion may have a propeller-like shape to generate a vortex in the reagent. Further, the protrusion portion 234 may comprise a plurality of specifically-shaped protrusions protruding from the body portion 232 or particle securing portion 233 of the magnet cover 230. Alternatively, the protrusion portion 234 may be provided by forming a plurality of specifically-shaped recesses on the body portion 232 or the particle securing portion 233 such that the rest of the body portion or the particle securing portion protrudes relative to the recesses.

In the present invention, the magnet cover 230 is preferably formed of a material having high magnetic permeability and may be formed of a thermoplastic resin or a thermosetting resin. For example, the magnet cover 230 is preferably formed of a material selected from the group consisting of polycarbonate (PC), polystyrene (PS), polymethylmethacrylate (PMMA), cycloolefin copolymer (COC), polyethylene (PE), polyamide (PA), polypropylene (PP), polyphenylene ether (PPE), polyoxymethylene (POM), polyetheretherketone (PEEK), polytetrafluoroethylene (PTFE), polyvinylchloride (PVC), polyvinylidene fluoride (PVDF), polybutylene terephthalate (PBT), fluorinated ethylene propylene (FEP), perfluoralkoxyalkane (PFA), acrylonitrile-butadiene-styrene (ABS), and combinations thereof, and is more preferably formed of polycarbonate (PC) or cycloolefin copolymer (COC).

A typical microcomputer may be used as a controller controlling movement of the aforementioned components. It will be understood that a microchip or the like that performs the same function may also be used. Such a controller is well known in the art and detailed description thereof will be omitted. The apparatus according to the present invention may be operated fully automatically by the controller.

Another aspect of the present invention relates to a method for transferring magnetic particles from a first chamber to a second chamber using the apparatus set forth above.

The method comprises:
providing a reagent comprising magnetic particles tein the first chamber;
positioning a transfer unit on the first chamber, the transfer unit comprising a magnet portion movable up and down in an axial direction thereof, and partially or entirely formed of a magnet, an outer ring potion movable up and down and between the chambers and allowing the magnet portion to move along a center thereof, and a magnet cover detachably coupled to the outer ring portion and preventing contamination of the magnet;
immersing a portion of the magnet cover in the reagent by moving the transfer unit downward with the magnet portion moved upward;
stirring the reagent in the first chamber through rotation, vertical movement, or rotation and vertical movement of the magnet cover;
securing the magnetic particles to a surface of the magnet cover by moving the magnet portion downward; and
moving the transfer unit having the magnetic particles secured thereto to the second chamber.

The method may further comprise, after moving the transfer unit to the second chamber, releasing the secured magnetic particles by moving the magnet portion upward.

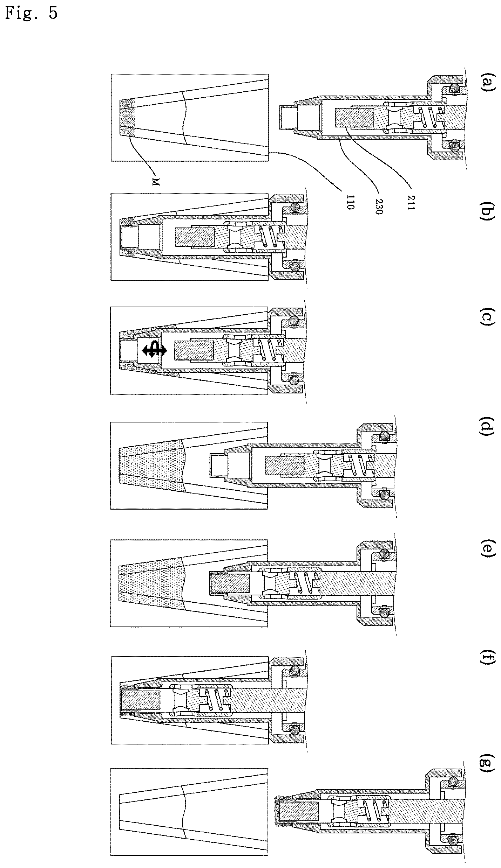

Now, a process of moving the magnetic particles from the first chamber to the second chamber using the apparatus according to the present invention will be described in detail with reference to FIG. 5.

First, the reagent containing a biological sample, a lysis buffer, and magnetic particles is provided to the first chamber 110, as shown in FIG. 5(a). Then, the biological sample is dissolved in the lysis buffer in the chamber 110 to discharge a target substance therefrom, and then the discharged target substance is coupled to the magnetic particles to form a complex. Therefore, the expression "moving the magnetic particles to another chamber using the transfer unit 200", as used herein, means not only moving the magnetic particles themselves, but also moving the complex of the magnetic particles and the target substance.

Referring to FIG. 5(a), the transfer unit 200 is positioned above the chamber 110 with the magnet cover 230 coupled to the outer ring portion 220. At this time, the magnet portion 210 is moved upward. The magnetic particles settle on a bottom of the chamber within a certain period of time after providing the reagent to the chamber 110.

Next, the transfer unit 200 is moved downward such that the lower end of the magnet cover 230 is in proximity to the bottom 112 of the chamber 110, as shown in FIG. 5(b). At this time, the magnet portion 210 remains moved up so as not to be completely inserted into the magnet cover 230.

Thereafter, when the outer ring portion 220 is operated to rotate and move up and down, as shown in FIG. 5(c), the magnet cover 230 rotates and moves up and down to stir the reagent. As a result, residual reaction further proceeds in the reagent and the magnetic particles are suspended in the reagent without adhering to the bottom or wall of the chamber 110. Here, the magnet cover 230 may be rotated at 100 rpm to 4,000 rpm, preferably 200 rpm to 2,000 rpm, and more preferably 250 rpm to 400 rpm, in order to optimize stirring of the reagent.

Here, it is important that the torque of the magnet cover 230 be applied uniformly the inside of the chamber to allow more efficient stirring of the reagent while preventing the magnetic particles from staying at the corners of the chamber. For this purpose, the wall 111 of the chamber is inclined at a predetermined angle with respect to a plane perpendicular to the bottom 112. Here, the predetermined angle preferably ranges from 3 to 10 degrees, more preferably 6 to 8 degrees.

Next, the transfer unit 200 is moved upward to allow the magnet cover 220 to be completely removed from the reagent, as shown in FIG. 5(d), and then the magnet portion 210 is moved downward to position the magnet 211 inside the particle securing portion 233 of the magnet cover 230, as shown in FIG. 5(e). The steps of FIG. 5(d) and FIG. 5(e) may be omitted, as needed. In this case, after the step of FIG. 5(c), the magnet portion 210 is moved downward to directly proceed to the step of FIG. 5(f).

FIG. 5(f) shows a state in which the magnet cover 230 is located in proximity to the bottom of the chamber by moving the transfer unit 200 downward, with the magnet portion 210 moved downward. It can be seen that the magnetic particles have been attracted and secured to the particle securing portion 233 by the magnetic force of the magnet 211, which is located inside the particle securing portion, unlike in FIG. 5(b).

After securing the magnetic particles to the magnet cover 230, the magnetic particles (having the target substance coupled thereto) are moved by moving the transfer unit 200 upward and then moving the transfer unit 200 to another chamber, as shown in FIG. 5(g). After moving the transfer unit 200 to the other chamber, the outer ring portion 220 is moved downward to introduce the magnet cover 220 and the magnetic particles secured thereto into a reagent in the other chamber and then the magnet portion 211 is moved upward such that magnetic force thereof is no longer applied to the magnetic particles, thereby allowing the magnetic particles to be released.

In the present invention, the biological sample may be at least one selected from the group consisting of blood, sputum, bacteria, semen, cell tissue, saliva, hair, urine, an aspirate sample, a swab sample, and a cell culture fluid, and the target substance may be nucleic acid.

In the present invention, the magnetic particles are preferably formed of a paramagnetic or superparamagnetic material, and preferably comprise an outer silica coating.

A further aspect of the present invention relates to a method for isolating or purifying a target substance from a biological sample using the apparatus according to the present invention.

The method comprises:
dissolving the biological sample and coupling the target substance to magnetic particles by providing a reagent, comprising the biological sample, the magnetic particles, and a lysis buffer to a first chamber;
positioning a transfer unit on the first chamber, the transfer unit comprising a magnet portion movable up and down in an axial direction thereof, and partially or entirely formed of a magnet, an outer ring portion movable up and down and between chambers and allowing the magnet portion to move along a center thereof, and a magnet cover detachably coupled to the outer ring portion and preventing contamination of the magnet;
immersing a portion of the magnet cover in the reagent by moving the transfer unit downward with the magnet portion moved upward;
stirring the reagent in the first chamber through rotation, vertical movement, or rotation and vertical movement of the magnet cover;
securing the magnetic particles having the target substance coupled thereto to a surface of the magnet cover by moving the magnet portion downward; and
washing the magnetic particles by moving the transfer unit, to which the magnetic particles having the target substance coupled thereto have been secured, to a second chamber containing a washing buffer; and
releasing the target substance from the magnetic particles by moving the transfer unit having the washed magnetic particles secured thereto to a third chamber containing an elution buffer.

The method may further comprise, after washing the magnetic particles, further washing the magnetic particles by moving the transfer unit having the washed magnetic particles secured thereto to a fourth chamber containing a second washing buffer.

In one embodiment, the method may further comprise washing the magnetic particles once or twice in the same manner as above.

Now, the method for isolating or purifying the target substance from the biological sample using the apparatus according to the present invention will be described in more detail.

In the present invention, the biological sample may be at least one selected from the group consisting of blood, sputum, bacteria, semen, cell tissue, saliva, hair, urine, an aspirate sample, a swab sample, and a cell culture fluid, and the target substance may be nucleic acid.

In the present invention, the magnetic particles are preferably formed of a paramagnetic or superparamagnetic material, and preferably comprise an outer silica coating.

In the present invention, the lysis buffer, the washing buffer, the second washing buffer, and the elution buffer may comprise any suitable buffers commonly used in the art, and may be prepared by appropriately using reagents disclosed in U.S. Patent No. 5,130,423, U.S. Patent No. 5,234,809, and International Patent Publication No. WO1991/012079.

For example, the lysis buffer according to the present invention may comprise: chaotropic salts such as GuHCl and GuSCN; buffers such as Tris-HCl; salts such as NaCl; chelators such as ethylenediaminetetraacetic acid (EDTA); and nonionic surfactants such as Tween-20 and Triton X-100.

For example, the washing buffer according to the present invention may comprise: chaotropic salts such as GuHCl and GuSCN; nonionic surfactants such as Tween-20 and Triton X-100; and alcohols such as ethanol.

For example, the second washing buffer according to the present invention may comprise: alcohols such as ethanol; and salts such as NaCl.

For example, the elution buffer according to the present invention may comprise: buffers such as Tris-HCl; and chelators such as EDTA.

In the present invention, dissolving the biological sample and coupling the target substance to the magnetic particles may be performed at a temperature of 40°C to 60°C, specifically 50°C to 58°C, more specifically about 56°C.

In another embodiment, dissolving the biological sample and coupling the target substance to the magnetic particles may comprise: dissolving the biological sample by providing the biological sample and the lysis buffer to the first chamber and heating the chamber to a temperature of 40°C to 60°C, specifically 50°C to 58°C, more specifically about 56°C; introducing ethanol and the magnetic particles into the first chamber containing the dissolved biological sample; and coupling the target substance to the magnetic particles.

In the present invention, upon moving a complex of the target substance and the magnetic particles into the third chamber, the target substance is released from the magnetic particles by the elution buffer in the third chamber. Although releasing the target substance from the magnetic particles may be performed at room temperature, it will be understood that releasing the target substance from the magnetic particles may be performed by heating the third chamber to a predetermined temperature depending upon the types of elution buffers and target substances.

After releasing the target substance from the magnetic particles, the transfer unit is moved downward to couple the magnetic particles to the magnet cover and is then moved to another chamber or to the outside to remove the magnetic particles from the third chamber such that only the target substance and the elution buffer remain in the third chamber. The target substance present in the elution buffer, for example, nucleic acid, may be isolated using any suitable method known in the art, and the isolated target substance, for example, the nucleic acid, may be used in a subsequent process such as a PCR process.

Although the present invention has been described with reference to some embodiments, in conjunction with the accompanying drawings, it should be understood that the foregoing embodiments are provided for illustration only and are not to be in any way construed as limiting the present invention, and that various modifications, changes, alterations, and equivalent embodiments can be made by those skilled in the art without departing from the spirit and scope of the invention.

## Claims

1. An apparatus for isolating or purifying a target substance from a biological sample using magnetic particles comprising:
a container comprising a plurality of chambers each having an opening at an upper side thereof; and
a transfer unit movable up and down to be introduced into the chambers, partially or entirely formed of a magnet to hold the magnetic particles by magnetic force, and movable between the chambers to transfer the magnetic particles between the chambers.

2. The apparatus according to claim 1, wherein the transfer unit comprises:
a magnet portion movable up and down in an axial direction thereof and partially or entirely formed of the magnet; and
an outer ring portion movable up and down and between the chambers and allowing the magnet portion to move along a center thereof.

3. The apparatus according to claim 2, wherein the transfer unit further comprises a magnet cover preventing contamination of the magnet.

4. The apparatus according to claim 3, wherein the magnet cover is detachably coupled to the outer ring portion.

5. The apparatus according to claim 4, wherein the magnet cover is coupled to the outer ring portion through press fitting.

6. The apparatus according to claim 4, wherein the outer ring portion is rotatably connected to a rotary motor and the magnet cover is rotatable by rotation of the outer ring portion.

7. The apparatus according to claim 6, wherein the outer ring and the magnet cover are rotated at 100 rpm to 1,000 rpm.

8. The apparatus according to claim 3, wherein the magnet cover is formed of a material selected from the group consisting of polycarbonate (PC), polystyrene (PS), polymethylmethacrylate (PMMA), cycloolefin copolymer (COC), polyethylene (PE), polyamide (PA), polypropylene (PP), polyphenylene ether (PPE), polyoxymethylene (POM), polyetheretherketone (PEEK), polytetrafluoroethylene (PTFE), polyvinylchloride (PVC), polyvinylidene fluoride (PVDF), polybutylene terephthalate (PBT), fluorinated ethylene propylene (FEP), perfluoralkoxyalkane (PFA), acrylonitrile-butadiene-styrene (ABS), and combinations thereof.

9. The apparatus according to claim 3, wherein the magnet cover comprises a protrusion portion comprising at least one protrusion.

10. The apparatus according to claim 2, wherein the magnet portion comprises the magnet and a support supporting the magnet.

11. The apparatus according to claim 10, wherein the magnet portion further comprises an elastic member between the magnet and the support.

12. The apparatus according to claim 1, wherein the magnet is a permanent magnet or an electromagnet.

13. The apparatus according to claim 1, wherein the plurality of chambers of the container is connected to one another in a row.

14. The apparatus according to claim 1, wherein an angle formed between a wall of each chamber and a plane perpendicular to a bottom of the chamber ranges from 3 to 10 degrees.

15. The apparatus according to claim 1, wherein the biological sample is at least one selected from the group consisting of blood, sputum, bacteria, semen, cell tissue, saliva, hair, urine, an aspirate sample, a swab sample, and a cell culture fluid.

16. The apparatus according to claim 1, wherein the target substance is nucleic acid.

17. The apparatus according to claim 1, wherein the magnetic particles are formed of a paramagnetic or superparamagnetic material.

18. The apparatus according to claim 1, wherein the magnetic particles comprise an outer silica coating.

19. A transfer device for transferring magnetic particles from a first chamber to a second chamber comprising:
a magnet portion movable up and down in an axial direction thereof and partially or entirely formed of a magnet; and
an outer ring portion movable up and down and between the chambers and allowing the magnet portion to move along a center thereof.

20. The transfer device according to claim 19, further comprising a magnet cover preventing contamination of the magnet.

21. The transfer device according to claim 20, wherein the magnet cover is detachably coupled to the outer ring.

22. The transfer device according to claim 21, wherein the magnet cover is coupled to the outer ring through press fitting.

23. The transfer device according to claim 21, wherein the outer ring is rotatable and the magnet cover is rotatable by rotation of the outer ring.

24. The transfer device according to claim 23, wherein the outer ring and the magnet cover are rotated at 100 rpm to 4,000 rpm.

25. The transfer device according to claim 20, wherein the magnet cover is formed of a material selected from the group consisting of polycarbonate (PC), polystyrene (PS), polymethylmethacrylate (PMMA), cycloolefin copolymer (COC), polyethylene (PE), polyamide (PA), polypropylene (PP), polyphenylene ether (PPE), polyoxymethylene (POM), polyetheretherketone (PEEK), polytetrafluoroethylene (PTFE), polyvinylchloride (PVC), polyvinylidene fluoride (PVDF), polybutylene terephthalate (PBT), fluorinated ethylene propylene (FEP), perfluoralkoxyalkane (PFA), acrylonitrile-butadiene-styrene (ABS), and combinations thereof.

26. The transfer device according to claim 20, wherein the magnet cover comprises a protrusion portion comprising at least one protrusion.

27. The transfer device according to claim 19, wherein the magnet is a permanent magnet or an electromagnet.

28. A method for transferring magnetic particles from a first chamber to a second chamber comprising:
providing a reagent comprising the magnetic particles to the first chamber;
positioning a transfer unit on the first chamber, the transfer unit comprising a magnet portion movable up and down in an axial direction thereof and partially or entirely formed of a magnet, an outer ring portion movable up and down and between the chambers and allowing the magnet portion to move along a center thereof, and a magnet cover detachably coupled to the outer ring portion and preventing contamination of the magnet;
immersing a portion of the magnet cover in the reagent by moving the transfer unit downward with the magnet portion moved upward;
stirring the reagent in the first chamber through rotation of the magnet cover, vertical movement of the magnet cover, or rotation and vertical movement of the magnet cover;
securing the magnetic particles to a surface of the magnet cover by moving the magnet portion downward; and
moving the transfer unit with the magnetic particles secured thereto to the second chamber.

29. The method according to claim 28, further comprising, after moving the transfer unit to the second chamber,
releasing the secured magnetic particles by moving the magnet portion upward.

30. The method according to claim 28 or 29,
wherein the reagent comprises a biological sample retaining a target substance and a lysis buffer,
wherein the biological sample is dissolved in the lysis buffer to discharge the target substance therefrom, and
wherein the discharged target substance is coupled to the magnetic particles.

31. The method according to claim 28, wherein the magnet cover is rotated at 100 rpm to 4,000 rpm.

32. The method according to claim 28, wherein the magnet cover is formed of a material selected from the group consisting of polycarbonate (PC), polystyrene (PS), polymethylmethacrylate (PMMA), cycloolefin copolymer (COC), polyethylene (PE), polyamide (PA), polypropylene (PP), polyphenylene ether (PPE), polyoxymethylene (POM), polyetheretherketone (PEEK), polytetrafluoroethylene (PTFE), polyvinylchloride (PVC), polyvinylidene fluoride (PVDF), polybutylene terephthalate (PBT), fluorinated ethylene propylene (FEP), perfluoralkoxyalkane (PFA), acrylonitrile-butadiene-styrene (ABS), and combinations thereof.

33. The method according to claim 28, wherein the magnet cover comprises a protrusion portion comprising at least one protrusion.

34. The method according to claim 30, wherein the biological sample is at least one selected from the group consisting of blood, sputum, bacteria, semen, cell tissue, saliva, hair, urine, an aspirate sample, a swab sample, and a cell culture fluid.

35. The method according to claim 30, wherein the target substance is nucleic acid.

36. The method according to claim 28, wherein the magnetic particles are formed of a paramagnetic or superparamagnetic material.

37. The method according to claim 28, wherein the magnetic particles comprise an outer silica coating.

38. A method for isolating or purifying a target substance from a biological sample using magnetic particles comprising:
dissolving the biological sample and coupling the target substance to the magnetic particles by providing a reagent comprising the biological sample, the magnetic particles, and a lysis buffer to a first chamber;
positioning a transfer unit on the first chamber, the transfer unit comprising a magnet portion movable up and down in an axial direction thereof and partially or entirely formed of a magnet, an outer ring portion movable up and down and between chambers and allowing the magnet portion to move along a center thereof, and a magnet cover detachably coupled to the outer ring portion and preventing contamination of the magnet;
immersing a portion of the magnet cover in the reagent by moving the transfer unit downward with the magnet portion moved upward;
stirring the reagent in the first chamber through rotation and/or vertical movement of the magnet cover;
securing the magnetic particles having the target substance coupled thereto to a surface of the magnet cover by moving the magnet portion downward; and
washing the magnetic particles by moving the transfer unit, to which the magnetic particles having the target substance coupled thereto have been secured, to the second chamber containing a washing buffer; and
releasing the target substance from the magnetic particles by moving the transfer unit having the washed magnetic particles secured thereto to a third chamber containing an elution buffer.

39. The method according to claim 38, further comprising, after washing the magnetic particles, further washing the magnetic particles by moving the transfer unit having the washed magnetic particles secured thereto to a fourth chamber containing a second washing buffer.

40. The method according to claim 38 or 39, wherein the magnet cover is rotated at 100 rpm to 4,000 rpm.

41. The method according to claim 38 or 39, wherein the magnet cover is formed of a material selected from the group consisting of polycarbonate (PC), polystyrene (PS), polymethylmethacrylate (PMMA), cycloolefin copolymer (COC), polyethylene (PE), polyamide (PA), polypropylene (PP), polyphenylene ether (PPE), polyoxymethylene (POM), polyetheretherketone (PEEK), polytetrafluoroethylene (PTFE), polyvinylchloride (PVC), polyvinylidene fluoride (PVDF), polybutylene terephthalate (PBT), fluorinated ethylene propylene (FEP), perfluoralkoxyalkane (PFA), acrylonitrile-butadiene-styrene (ABS), and combinations thereof.

42. The method according to claim 38 or 39, wherein the magnet cover comprises a protrusion portion comprising at least one protrusion.

43. The method according to claim 38 or 39, wherein the biological sample is at least one selected from the group consisting of blood, sputum, bacteria, semen, cell tissue, saliva, hair, urine, an aspirate sample, a swab sample, and a cell culture fluid.

44. The method according to claim 38 or 39, wherein the target substance is nucleic acid.

45. The method according to claim 38 or 39, wherein the magnetic particles are formed of a paramagnetic or superparamagnetic material.

46. The method according to claim 38 or 39, wherein the magnetic particles comprise an outer silica coating.
